(19) Europäisches Patentamt
European Patent Office
Office européen des brevets

(11) Publication numbe 0 034 407
B1

(12) # EUROPEAN PATENT SPECIFICATION

(45) Date of publication of patent specification: 07.09.83

(51) Int. Cl.³: C 07 C 9/04, C 07 C 1/20, B 01 J 23/74

(21) Application number: 81300221.9

(22) Date of filing: 19.01.81

(54) **Process for the manufacture of methane-containing gas from ethanol.**

(30) Priority: 31.01.80 GB 8003317

(43) Date of publication of application: 26.08.81 Bulletin 81/34

(45) Publication of the grant of the patent: 07.09.83 Bulletin 83/36

(84) Designated Contracting States: BE DE FR IT NL

(56) References cited:
GB - A - 1 432 696
GB - A - 1 447 974
GB - A - 1 494 029
US - A - 4 148 835
US - A - 4 182 926

(73) Proprietor: British Gas Corporation
Rivermill House 152 Grosvenor Road
London SW1V 3JL (GB)

(72) Inventor: Williams, Alan
40 Willow Drive Cheswick Green
Shirley West Midlands B90 4HW (GB)
Inventor: Banks, Reginald George Sinclair
55 St. Barnards Road
Olton West Midlands B92 79F (GB)

(74) Representative: Wallace, Walter
British Gas Corporation Patents Department 326
High Holborn
London, WC1V 7PT (GB)

EP 0 034 407 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Process for the manufacture of methane-containing gas from ethanol

This invention relates to the manufacture of methane-containing gas from ethanol and in particular to the manufacture of gas which may be used as a substitute for natural gas.

The realisation that there is not an inexhaustible supply of fossil fuels has awakened interest in 'renewable energy sources', for instance biologically derived fuels. One such fuel is ethanol, manufactured by fermentation of carbohydrates derived from cereal and sugar crops, and already some countries are producing substantial quantities for use as motor fuel. Conceivably, the production of ethanol might increase to the extent that it would become available not just as a liquid fuel but for use as a feedstock from which fuel gases could be manufactured.

A gas manufacturing process best suited to this purpose would be one which produced a methane-containing gas with a sufficiently high methane content to allow its ready conversion into substitute natural gas (SNG). This is because natural gas, itself a fossil fuel, is so widely used and because it is associated with a well developed technology, both for its utilisation and its processing. Other fuel gases, or synthesis gases if they were required, could be produced from the methane-containing gas by known methods.

The best known process for the manufacture of SNG is the CRG process (described in UK Patent Specification No. 820,257) which, though it has been developed over the years to gasify hydrocarbons ranging from LPG to gas oil, evolved from experimental work on a peak load process intended for use with synthetic methanol as a feedstock. This process is described in our prior UK Patent Specification No. 1447974 wherein *inter alia* a mixture of methanol vapour and steam at a temperature of at least 300°C is passed into a bed of nickel catalyst and at least a portion of the methane-containing product gas is recirculated and admixed with the mixture of methanol vapour and steam. The temperature of the steam-methanol mixture and the amount of product gas recirculated is chosen such that the temperature of the catalyst bed is maintained at between 400 and 500°C. Consequently, this process appeared to offer the prospect that ethanol could be substituted for hydrocarbon as feedstock. Assuming satisfactory catalyst performance, calculations showed that typical conditions used in the gasification of light hydrocarbons would be quite acceptable with ethanol. Some reduction in overall thermal efficiency would result but efficiency would still be comparable with that of the process when using heavier hydrocarbons. The underlying assumption, that the catalyst would give products at equilibrium as it does with hydrocarbon feedstock and that it would have an adequate working life, therefore required experimental verification.

When the experiments were carried out, they showed, quite unexpectedly, that attempting to gasify ethanol over CRG catalyst using typical conditions for gasifying hydrocarbons very rapidly leads to carbon deposition on the catalyst and blockage of the reaction tube.

Catalytic process for gasifying ethanol are known. For example US Patent Specification No. 4148835 describes the production of olefins by the gasification of $C_1$—$C_4$ monohydric alcohols, e.g. ethanol over a catalyst comprising a crystalline aluminosilicate zeolite. Reference to the Example will show that although methane is produced, it is only produced in very low yield and is a by-product with olefines being the major product. Such a process would have no utility for the production of methane containing gases on a commercial scale since the zeolite catalyst employed are essentially dehyrogenation catalysts and not hydrogenation/reforming catalysts.

The present invention is based on the observation that a mixture of ethanol and steam can be made to react over a nickel-alumina catalyst without rapidly depositing carbon provided the highest temperature reached over the catalyst does not exceed 450°C and that the products then obtained are at equilibrium and rich in methane.

Accordingly, this invention provides a process for the manufacture of methane-containing gas from ethanol, wherein a mixture of ethanol vapour and steam at a temperature not exceeding 350°C is passed at atmospheric or superatmospheric pressure through a bed of nickel-alumina catalyst and the maximum temperature within the catalyst bed is maintained below 450°C.

The catalysts used in the process of the invention can be those known and used for the steam reforming of hydrocarbon feedstocks. Preferably the catalyst will be a coprecipitated catalyst such as those described in UK Patent Specification Nos. 1,525,017 and 1,550,749 and EPC Patent Specification No. 78 300131.6.

Although the process of the invention resembles the CRG process and can make use of the same catalysts, there is the difference that ethanol, unlike a hydrocarbon feedstock, does not consume steam to any great extent. Indeed no steam would be required if ethanol could be made to react according to the equation

$$2C_2H_5OH \rightleftharpoons 3CH_4 + CO_2 \quad (1)$$

without interference from side reactions and carbon deposition. This reaction is strongly exothermic, however, and without steam the

temperature rise in the catalyst bed would be substantially greater and carbon deposition unavoidable. To prevent carbon deposition it has been found necessary both to limit the heat input by using low catalyst inlet temperatures and to absorb the reaction heat using a substantial proportion of steam.

The catalyst inlet temperature to be used will depend on the activity of the catalyst and on the process conditions; it must be high enough to initiate reaction over the catalyst and to maintain the mixture of ethanol vapour and steam in the vapour state but not so high that a wasteful quantity of steam must then be used to limit the temperature rise over the catalyst. A practical upper limit is 350°C. At this temperature it is possible to avoid carbon deposition without using grossly inefficient steam/ethanol ratios. It is preferred, however, to use catalyst inlet temperatures ranging from 250—325°C, typically about 300°C.

The preferred range of steam/ethanol ratios is from 1.75 to 2.25 parts by weight of steam to one part by weight of ethanol. In combination with the preferred range of catalyst inlet temperatures, this range allows the maximum temperature in the catalyst bed to be maintained below 450°C at pressures that are commonly used in gas manufacture and at comparable thermal efficiency. Moreover, although the product gas will contain hydrogen, its composition will not be far removed from that indicated by equation (1) as is shown in the accompanying example. This is particularly advantageous in the manufacture of SNG.

When ethanol reacts in the presence of steam over a nickel-alumina catalyst such as CRG catalyst, the product gas obtained is at equilibrium with respect to the reactions.

$$CH_4 + H_2O \rightleftharpoons CO + 3H_2 \quad (2)$$

$$CO + H_2O \rightleftharpoons CO_2 + H_2 \quad (3)$$

as in the CRG process itself. Consequently, much of the process technology associated with the CRG process is expected to be applicable to ethanol gasification. Thus fuel gases of lower methane content, synthesis gas or hydrogen could be produced by re-establishing equilibrium at higher temperatures in subsequent catalytic stages. Alternatively, a lower temperature stage or stages, followed by carbon dioxide removal, would give virtually pure methane, which may be enriched with LPG to produce SNG.

Elaboration of the process by gasifying ethanol in more than one stage is considered to be within the scope of the invention. Using two stages, for example, the wet product gas from the first stage would be cooled and mixed with more ethanol vapour before entering the second stage. As in the first stage, the catalyst inlet temperature would need to be high enough to initiate reaction over the catalyst and maintain the steam and ethanol vapour in the vapour state but low enough to avoid carbon deposition. The benefit of two-stage operation would be a more economical use of steam because part of the reaction heat in the second stage would be absorbed by the product gas from the first, allowing a lower overall steam/ethanol ratio to be used.

Recirculation of product gas is a well-known technique which, it is recognised, could be used with the process of the invention to limit the temperature rise in the catalyst bed.

Example

The experimental apparatus comprised a stainless steel reactor mounted vertically in an electric furnace and containing 3 mm cylindrical pellets of nickel-alumina catalyst, which, before use, was reduced at 450°C in hydrogen for 16 hours. To minimise heat loss during the experiment, the furnace controls were adjusted to give a uniform temperature down the catalyst bed with no reactants flowing. No further adjustments were then made. Provision was made to evaporate and preheat a mixture of water and ethanol, the vaporised mixture then being passed downwards over the catalyst, and to condense steam from the product gas. Pressure was maintained by a controller at the gas outlet.

The experimental conditions used for this example are as shown in Table 1. As can be seen, a product gas of high methane content was obtained over a period exceeding a thousand hours. There was some increase in reaction zone length, most of which occurred within the first 100 hours, indicating only slow deactivation of the catalyst. No signs of carbon deposition were observed. The experiment was terminated voluntarily and examination of the discharged catalyst showed that it had changed little in use.

TABLE 1

| | |
|---|---|
| Pressure, (Bars gauge) | 31.0 |
| Preheat temperature, °C | 300 |
| Steam/ethanol (by weight) | 2 |
| LHSV, vol/vol. hour (of mixture) | 15 |
| Properties of ethanol used | |
| specific gravity | 0.7904—0.7935 |
| ethanol content, % vol | 99.4—100 |
| sulphur content, ppm wt | less than 0.2 |
| Catalyst | Commercial catalyst* |
| Catalyst size, mm × mm | 3 × 3 |
| Catalyst bed depth, cm | 83.82 |
| Duration of experiment, hours | 1010 |
| Initial reaction zone length, cm | 19.3 |
| Final reaction zone length, cm | 48.25 |
| Maximum temperature reached, °C | 405-409 |
| Product gas composition, % vol (dry basis) | |
| methane | 68.6 |
| hydrogen | 7.3 |
| carbon dioxide | 24.1 |
| carbon monoxide | not detectable |

*The catalyst is described and claimed in UK Patent Specification No. 1,525,017

**Claims**

1. A process for the manufacture of methane-containing gas from ethanol, wherein a mixture of ethanol vapour and steam at a temperature not exceeding 350°C is passed at atmospheric or superatmospheric pressure through a bed of nickel-alumina catalyst and the maximum temperature within the catalyst bed is maintained below 450°C.

2. A process as claimed in Claim 1, in which the mixture of ethanol vapour and steam is at a temperature ranging from 250—350°C.

3. A process as claimed in Claim 1 or Claim 2, in which the mixture of ethanol vapour and steam contains from 1.75 to 2.25 parts by weight of steam to one part by weight of ethanol.

4. A process as claimed in any of the preceding claims in which the nickel-alumina catalyst is a coprecipitated nickel-alumina catalyst.

5. A process as claimed in any of the preceding claims in which two catalytic stages are used, the wet product gas from the first stage being cooled and mixed with more ethanol vapour before entering the second stage.

6. A process as claimed in any of the preceding claims in which product gas is recirculated through the catalyst bed.

**Revendications**

1. Procédé de production d'un gaz contenant du méthane à partir d'éthanol, dans lequel on fait passer un mélange de vapeur d'éthanol et de vapeur d'eau, à une température n'excédant pas 350°C, à la pression atmosphérique ou à une pression supérieure à la pression atmosphérique à travers un lit de catalyseur nickel-alumine et on maintient la température maximale dans le lit de catalyseur au-dessous de 450°C.

2. Procédé suivant la revendication 1, dans lequel le mélange de vapeur d'éthanol et de vapeur d'eau est à une température comprise entre 250 et 350°C.

3. Procédé suivant la revendication 1 ou la revendication 2, dans lequel le mélange de vapeur d'éthanol et de vapeur d'eau contient 1,75 à 2,25 parties en poids de vapeur d'eau pour une partie en poids d'éthanol.

4. Procédé suivant l'une quelconque des revendications précédentes, dans lequel le catalyseur nickel-alumine est un catalyseur nickel-alumine coprécipité.

5. Procédé suivant l'une quelconque des revendications précédentes, dans lequel on utilise deux étages catalytiques, le gaz humide produit venant du premier étage étant refroidi et mélangé avec une quantité additionnelle de vapeur d'éthanol avant d'entrer dans le second étage.

6. Procédé suivant l'une quelconque des revendications précédentes, dans lequel le gaz produit est recyclé à travers le lit de catalyseur.

**Patentansprüche**

1. Verfahren zur Herstellung von Methan enthaltendem Gas aus Äthanol, dadurch gekennzeichnet, daß ein Gemisch aus Äthanoldampf und Wasserdampf mit einer 350°C nicht überschreitenden Temperatur bei atmosphärischem oder überatmosphärischem Druck durch ein Katalysatorbett aus Nickel-Aluminiumoxid geleitet wird und daß die Maximaltemperatur innerhalb des Katalysatorbetts unterhalb 450°C gehalten wird.

2. Verfahren nach Anspruch 1, dadurch gekennzeichnet, daß das Gemisch aus Äthanoldampf und Wasserdampf eine Temperatur im Bereich von 250—350°C aufweist.

3. Verfahren nach Anspruch 1 oder 2, dadurch gekennzeichnet, daß das Gemisch aus Äthanoldampf und Wasserdampf 1,75 bis 2,25 Gewichtsteile Wasserdampf zu einem Gewichtsteil Äthanol enthält.

4. Verfahren nach einem der Ansprüche 1 bis 3, dadurch gekennzeichnet, daß der Nickel-Aluminiumoxid-Katalysator ein mitgefällter Nickel-Aluminiumoxid-Katalysator ist.

5. Verfahren nach einem der Ansprüche 1 bis 4, gekennzeichnet durch die Anwendung zweier katalytischer Stufen, wobei das nasse Produktgas aus der ersten Stufe gekühlt und mit mehr Äthanoldampf gemischt wird, bevor es in die zweite Stufe gelangt.

6. Verfahren nach einem der Ansprüche 1 bis 5, dadurch gekennzeichnet, daß Produktgas durch das Katalysatorbett hindurch rückzirkuliert wird.